# EUROPEAN PATENT SPECIFICATION

(11) **EP 1 558 095 B1**
(45) Date of publication and mention of the grant of the patent: **14.06.2006**
(21) Application number: 03759163.3
(22) Date of filing: 04.11.2003
(51) Int. Cl.: A23L 1/29, A61K 35/20

(54) **A FEED OR FOOD PRODUCT COMPOSITION**
FUTTER- ODER NAHRUNGSMITTELZUSAMMENSETZUNG
COMPOSITION D'ALIMENT POUR ANIMAUX OU DE PRODUIT ALIMENTAIRE

(30) Priority: 06.11.2002 SE 0203265
(43) Date of publication of application: 03.08.2005
(73) Proprietor: Coloplus AB, 211 43 Malmö (SE)
(72) Inventor: ELFSTRAND, Lidia, S-217 45 Malmö (SE); FLOREN, Claes-Henrik, S-224 57 Lund (SE); HAGMAN, Conny, S-217 41 Malmö (SE); IHSE, Ingemar, S-224 74 Lund (SE)
(74) Representative: Karlsson, Leif Gunnar Börje
(86) International application number: PCT/SE2003/001696
(87) International publication number: WO 2004/041004

(56) References cited:
- WO-A1-97/05884
- WO-A2-02/47612
- US-A- 5 531 989
- US-B2- 6 410 058

## Description

The present invention relates to a feed or food product composition. More specifically, the invention relates to a feed or food product composition, which especially is adapted to deliver a mixture of bioactive bovine colostrum components to the digestive tract of a mammal.

Colostrum is a pre-milk fluid secreted directly for up to 72 hours after birth by every mammal. Bovine colostrum consists of a mixture of lacteal secretions and constituents of blood serum, notably immunoglobulins and other serum proteins, which accumulate in the mammary gland. Its composition is quite different from that of ruminant milk in established lactation. Thus, colostrum is a very complex fluid rich in nutrients, antibodies and growth factors and is characterized by its very high level of several other bioactive components.

In contrast to milk, colostrum is not constantly produced by the cow but only at the birth of the calf. Then it is produced in excess and only partly consumed by the calf. The remaining portion can then be collected. Thus, colostrum is a natural food.

True colostrum is defined as milk from the first three milkings or milk collected during the first 24 hours after parturition. If the collection of colostrum does not take place shortly after birth, the bioactive components in colostrum will rapidly deteriorate with time. Whole colostrum can be fractionated and utilized as separate components.

Research has shown that bovine colostrum can work effectively in humans as well as other mammals because of the reciprocal compatibility of the components therein.

Bovine colostrum has several established medical effects. The main natural function of the colostrum immunoglobulins is supposed to provide the calf with passive immunity. It has been suggested that oral administration of colostrum immunoglobulin concentrates of bovine origin could be effective in preventing diarrheal diseases such as infantile rotavirus gastroenteritis, HIV-associated diarrhoea or travelers' diarrheoa, which affects about half of all travelers to developing countries. In animals and humans, IgG₁ of bovine origin has also been shown to passively protect against infection by various enteric pathogens.

Furthermore, bovine colostrum supplementation has been shown to increase the serum concentration of IGF-1 in athletes during training and an anti-aging effect of bovine colostrum extract has also been reported.

Colostrum, produced after immunization, contains a high concentration of specific antibodies and can be used in prevention of various enteric diseases. Many clinical studies have been carried out to demonstrate the efficacy of using immune milk and colostrum preparations.

Immune colostrum-based test materials have also been proven effective in prophylaxis against specific various infectious diseases in humans. Good results have been obtained with test materials targeted against rotavirus, Shigella flexneri, Escherichia coli, Clostridium difficile, Streptococcus mutans, Cryptosporidium parvum and Helicobacter pylori. Some successful attempts have been made to use immune milk in balancing gastrointestinal microbial flora.

Colostrum products can be manufactured from whole colostrum, defatted colostrum, whey colostrum and Ig-concentrate. Many manufacturers add excipients, emulsifiers, or other chemicals in order to obtain a better dispersability of colostrum in water.

Liquid colostrum products for dairy products are shown in US 6,202,546 and US 6,248,366, suitable dairy products being different kinds of beverages. In US 6,410,058 a dietary supplement composition for a mammal is shown, which comprises lactoferrin and colostrum, and silicon dioxide and/or magnesium stearate in the nanometer range as a physical binder. The composition is designed for an effective absorption through the lining of the oral cavity so that lactoferrin and colostrum achieve their optimal effect when dissolved slowly in the mouth, rather than being swollowed directly. Modified citrus pectin can be included in the composition as an optional component. The polysaccharide has been treated to reduce its molecular size, thereby increasing its solubility and ability to be absorbed into the blood stream.

However, liquid colostrum products suffer from the same deterioration problem as the natural colostrum. The bioactivity of the colostrum components declines in dependence of the treatment, especially in the presence of water. In addition, the bioactive components also partly lose their bioactivities in all steps of a separation process and during a subsequent drying since the heat used in the drying processes and the pasteurisation reduces the colostrum bioactivities. Furthermore, the bioactive components are more thermal labile after subsequent removal of fat and other colostrum components.

When the colostrum is ingested as a liquid by a mammal with one stomach, e.g. a human being, it will empty relatively quickly from the stomach, the kinetics of liquid emptying following first order kinetics.

Under normal fed state conditions, the stomach empties only liquids and suspended finely particulate solids. In the distal part of the stomach the particle size of ingested solids is reduced to a fluid-like consistency. After this is accomplished, the material produced is emptied as a liquid suspension.

The gastric emptying during the fed state involves a complex interaction between gastric peristalsis, pyloric sphincter function and feedback information from the duodenum. As the peristaltic waves approach the pylorus, they increase in speed and strength. Only a thin stream of liquid escapes into the duodenum before the pylorus contracts and occludes the lumen. The material delivered to the duodenum consists of gastric secretions, digested and solubilized portions of a meal, and solid particles of food. These particles of food are driven against the closed pylorus by the rapidly advancing peristaltic wave and sheared as they are jetted back to the proximal stomach when the peristaltic wave approaches the closed pylorus. This process continues until all digestible food is discharged into the duodenum.

The object of the invention is to provide a feed or food product composition, which comprises bioactive bovine colostrum components as well as nutritives, whereby the above-mentioned problems are eliminated.

Another object of the invention is to provide a feed or food product composition, whereby the internal transit time through the digestive tract of a mammal can be prolonged and controlled.

Still another object is to provide a feed or food product composition, whereby the survival and resistance of the bioactive colostrum components in the gastrointestinal tract is increased and the components are protected against acid denaturation in the stomach as well as alkaline intestinal denaturation.

A further object of the invention is to provide a feed or food product composition, whereby the contact time of the bioactive colostrum components with mucosal surfaces is increased, the bioactivity, e.g. local immune responses, also being increased.

Yet a further object is to provide a feed or food product composition, whereby the high buffering capacity of natural colostrum is maintained.

Still yet a further object is to provide a feed or food product composition, which as a dry product is suitable for storing, and which subsequently can be mixed with an aqueous liquid to a consistency suitable for intake by for example a human being. The composition should be chemically as well as biologically stable during production and storage with preserved activity, also in a final product.

Still yet another object is to provide a feed or food product composition, which fulfils all the requirements of the National Food Administration.

These objects are accomplished by the feed or food product composition according to the invention as claimed.

According to the invention, the feed or food product composition to be delivered to the digestive tract of a mammal comprises a mixture of bioactive bovine colostrum components as well as nutritives from organic particulate matter.

In this connection the term "bioactive components", present in bovine colostrum, encompasses immunmoglobulines, growth factors, and antibacterial peptides, which are obtained in high concentrations in the colostrum.

There are three classes of immunoglobulins in bovine colostrum (IgG, IgA and IgM). The vast majority of total immunoglobulins (Igs) present in the mammary secretions of ruminant species (bovine) are transported from the blood. The blood Igs are overwhelmingly IgG, specifically of the IgG₁ subclass. As any foreign protein, bovine IgG may represent a potent antigen and stimulate production of antibodies. Bovine IgG is thus - apart from its antigen-specific mechanisms - able to interact with and influence the human immune system.

Not only immunoglobulins but also cell-specific antibodies and other immune factors protect against viruses, bacteria, allergens, toxins and yeast are considered as bioactive bovine colostrum components. These components have several effects on the immune system of a mammal and can improve its immune defense.

The major growth factors in bovine colostrum are transforming growth factor-βs (TGF-β1 and TGF-β2) and insulin-like growth factors (IGF-1 and IGF-2). These factors may play an important role, especially in the growth and development of the gastrointestinal tract of newborn animals.

*In vivo*, IGF-1 and IGF-2 are supposed to act both as endocrine hormones via the blood and locally as paracrine and autocrine growth factors. The major IGF form in bovine colostrum, IGF-1, has been shown to stimulate cell proliferation in the GI-tract of newborn piglets and calves and may have effects on mammary tissue.

Likewise, the term "bioactive components", present in bovine colostrum, encompasses colostral proteins and peptides, such as α-lactalbumin, β-lactoglobulin, lactoferrin, lysozyme, lactoperoxidase, glucomacropeptide, and other growth factors. These components been implicated in a number of biological effects observed in human as well as animal studies. For example, the growth factors stimulate especially the growth of the gut mucosa.

An important aspect of the invention is that the term "bioactive components" also encompasses fat in the sense that it has a hormonal and/or retarding effect on the gastric emptying. The fat can originate from bovine colostrum or from another edible source.

Thus, in the inventive composition the fat content can be used for modifying the rate, by which bioactive bovine colostrum components leave the stomach. The opening of the pylorus is inhibited and delayed by fat, and an increase in fat content thus results in a retarding effect. A high fat content not only results in a longer residence time in the stomach but also in the intestine by slowing the gastrointestinal movements.

Accordingly, the colostrum should be collected and processed in such a way that its bioactive components, including its fat content, are well preserved. Such a procedure comprises separation of fat, pasteurization and pulverization. By using the entire colostrum system without further fractionation additional components, such as carbohydrates, proteins and salts, can protect the more sensitive components of colostrum from inactivation during the conventional processing in the diary industry.

Preferably, the colostrum is frozen and stored at least at -18°C within 1 h from milking.

In order to exert its effect, the fat content of the feed or food product composition should be between 5 and 20 wt% on dry weight basis.

The fat is preferably bovine colostrum fat originating from the pre-separated fat, which should be non-homogenised fat. However, the fat can also be vegetable fat. Suitable vegetable fat is obtained from for example rice, soy, rape, palm, and maize. Of course, the fat can be a mixture of animal and vegetable fat.

The fat also protects the immunoglobulins and growth factors from a negative exposure in the intestinal environment. It is believed that colostrum as an emulsion interacts with the particles, which in turn also protect the bioactive components.

According to the invention, the feed or food product composition to be delivered to the digestive tract of a mammal also comprises organic particulate matter. The size and amount of the particulate matter in the inventive composition is used to control the time required to empty the composition from the stomach, since small particles influence the gastric emptying to a lesser degree.

In this connection organic particulate matter means solid or semi-solid matter that - when entering the digestive tract - influences the opening of the pylorus. The organic particulate matter has a size of 0.3-7 mm in diameter. These particles are then reduced in size with time during its mechanical treatment in the stomach, thereby allowing a slower intestinal transport time than for milk or pure colstrum only.

It is believed that a "carrier" of organic particulate matter encloses the bioactive components of colostrum and protect them from a rapid disintegration in the stomach as well as prolong the period for their release.

The opening of the pylorus can also be manipulated by varying the content of the particulate matter. A low content of particulate matter results in a more fluid-like consistency of the material obtained from the distal stomach. When a high content of particulate matter is processed, having a large particle size, the viscosity of the suspension obtained is increased, and the gastric emptying rate is slowed.

The particulate matter of an edible material, e.g. crumbs, pellets, etc., is equally distributed in the composition. Its content in the feed or food product composition should be between 40 and 80 wt% on dry weight basis.

The organic particulate matter can be obtained directly from a producer on the market with a specified particle size designed for a selected application of the invention. This is important as optimal particle size may differ in products aimed for adult or for babies. For example, banana flakes are in one case obtained from Chiquita, which have a particle size of 2x2 mm

Preferably, the particulate matter is particulate insoluble dietary fibers, such as for example cellulose, rice, maize, and soy fibers in particulate form, or mixtures thereof, which are generally only slightly digested by the intestinal flora. In this connection dietary fibers are those food fiber ingredients which are not digested by human digesting enzymes. The dietary fibers of the composition should be as tasteless and odorless as possible in order not to negatively influence the final products.

Generally, the particulate dietary fibers used are those produced in a spherical to rod-like shape. Their water retaining capacity is reduced with size. Preferably, the water retaining capacity of the particulate fibers should not exceed 10 times the dry weight of the final product in order not to affect the texture of the particles and thus their efficiacy.

In a preferred embodiment the composition according to the invention is in a dry state that adapted to be mixed with a liquid, e.g. water or milk, preferably water, to form a product of suitable consistency for ingestion by a mammal. Although "dry", the inventive composition contains some water (less than 5 wt%). However, this water is bound water and does not negatively influence the bioactive bovine colostrum components during storage. An aqueous liquid can be added to the composition in its dry state up to 10 times its dry weight with retained effect. Of course, the amount of water depends on the amount of particulate matter in the composition.

When water has been added to the composition, an environment is provided for maintaining optimal form of delivery of bioactivity. The buffering capacity of colostrum as well as that of the particulate dietary fibers assist in withstanding the acid pH of the stomach and subsequently escaping the digestion of the bioactive components in the small intestine. The pH of the feed or food product composition in a wet state should be between 5 and 7, preferably the same pH as colostrum, i.e. pH 6.3.

Colostrum of good quality should have more than 60 mg immunoglobulins/ml, the immunoglobulin content being used as a marker. IgG is the major bovine immunoglobulin, and IgG₁ represents 90% or more of the total immunoglobulins in colostrum. In the feed or food product composition according to the invention, the bioactive bovine colostrum components should comprise 10-40 wt% bovine colostrum powder of 20-25 wt% IgG on dry weight basis. The immunoglobulins can be IgG, IgA, IgM, IgE, and IgY.

The daily intake of bioactive bovine colostrum components by a human being, preferably given twice or thrice a day, should be 10.5 g colostrum powder/day/person. This optimum colostrum content corresponds to about 2.1 to 8.0 g IgG per person and day, which should be independent of the fat content of the composition.

The inventive composition may be supplemented with further non-colostrum components, for example nutritionally effective amounts of proteins, carbohydrates, vitamins and minerals. Milk proteins as whey proteins from bovine milk can be used as consistency agent. The nutritional benefits of whey proteins can be utilised by increasing the protein content and balance the amino acid profile of the feed or food product composition.

Likewise, water-soluble polysaccharides, starch polysaccharides and maltodextrines, from maize, potatoes, corn, and wheat, as well as low molecular weight carbohydrates can be included in the inventive composition. Additional immunoglobulins can also be included, i.e. IgG from whey and IgY from eggs.

The inventive feed or food product composition is intended to be used as a functional food product. Preferably, such a product is a "porridge" product or a patty. A porridge product should per portion of about 35 g contain at least 5.25 g (15 %) colostrum powder of 20 % IgG₁. It is an advantage if such a product has a high fiber bulk and is devoid of easily absorbed carbohydrates. A patty product can comprise 30 % fat and 55 % proteins as well as flavouring agents.

In a preferred embodiment of the invention the composition essentially consists of four parts:
1. An active part of colostrum, including colostrum fat, as a powder, which has been collected and processed so that its bioactivity is preserved.
2. A "carrier"; dry rice and banana particles with a size of 0.3-7 mm.
3. Thickener/flavouring agents; maltodextrines, sugar, and whey proteins.
4. Optional vegetable fat.

### EXAMPLES

The feed or food product composition according to the invention is especially beneficial for patients with gastro-intestinal symptoms, where bacterial overgrowth is the cause of the symptoms.
Examples are:
- Patients with chronic HIV-associated diarrhoea.
- Patients with irritated large intestine (colon irritable), the symptoms being dominated by rumblings, pains and gas tensions;
- Patients with dyspepsia, the symptoms being dominated by pains, indisposition, and bloatedness;
- Diabetics, who after long duration of the disease and insulin treatment develope altered gastric and intestinal motility; and
- Patients carrying Heliobacter pylori.

### Example 1.

Thus, the inventive composition has been included in a study to investigate if colostrum administered as a food product beneficially influences the severe diarrhoea associated with HIV infection with and without manifest AIDS.

### Exclusion criteria

Patients 18 years of age or older with verified HIV infection with and without manifest AIDS and stool frequency of 4 or more per 24 hours.

The test substance was the preferred embodiment of the inventive composition (c.f. above).

### Design of the study

The study is an open, non-randomized pilot study. As no similar, previous studies are available no power calculations are possible. A number of 30 patients is estimated to be appropriate in this initial study and they will act as their own controls.

The test substance is packed in portions of 35 g. Each portion contains 3-4 g of immunoglobulin and is by the patient mixed with 1.25 dl of tepid water (<20°C) just before take in. The test substance, which thus has the characteristics of porridge, is taken twice a day, with the first and last meal, respectively. It should be stored in refrigerator at a temperature ≤4-6°C.

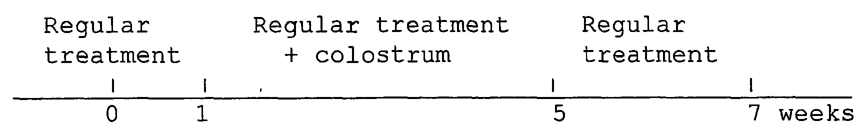

Weeks 2-5 the colostrum food product is given twice daily according to instructions. At the start of the study stool microscopy is done and blood samples taken for CD4 test. Weeks 0-7 the following parameters are studied and documented:
- frequency of stools per 24 hours;
- S-hemoglobin in end of week 1, 5 and 7;
- hematocrite in the end of week 1, 5 and 7;
- S-albumin in the end of week 1, 5 and 7;
- body weight in the end of week 1, 5 and 7;
- body mass index in the end of week 1, 5 and 7;and
- fatigue according to the VAS-scale in the end of week 1, 5 and 7.

Data will be analysed and statistical differences calculated.

### Example 2.

The effects of a colostrum food product according to the invention on chronic intestinal symptoms were also studied.

The studies are double blind studies comparing bovine milk and bovine colostrum and are carried out on three patient categories as below:
1. Patients with bloating, pain and diarrhoea, where the symptoms are supposed to arise from the small and large intestine (irritable bowel syndrome).
2. Patients with dyspepsia, where the symptoms are considered to arise from the stomach and the proximal small intestine (dyspepsia).
3. Patients with long standing diabetes mellitius and disturbances in the intestinal motility (diabetic gastric paresis).

For all patient categories the concept is that motility disturbances will give rise to a pathological flora, which will give the mentioned symptoms. The content in colostrum of immune factors and lactoferrin will decrease the growth of the pathological flora and any harm done to the intestine could be repaired by growth hormones in colostrum.

The preliminary results from the studies are very encouraging with reference to the effects of the inventive colostrum food product on chronic HIV-associated diarrhoea.

## Claims

1. A feed or food product composition to be delivered to the digestive tract of a mammal, which comprises a mixture of bovine colostrum, comprising bioactive components, and organic particulate matter having a size between 0.3 and 7 mm in diameter.

2. The feed or food product composition as in claim 1, wherein the content of said organic particulate matter is between 40 and 80 wt% on dry weight basis.

3. The feed or food product composition as in claim 1, wherein said organic particulate matter is particulate insoluble dietary fibers.

4. The feed or food product composition as in claim 3, wherein said particulate insoluble dietary fibers are particulate fibers of cellulose, rice, maize, or soy, or a mixture thereof.

5. The feed or food product composition as in claim 3, wherein said particulate insoluble dietary fibers have a water retaining capacity of up to 10 times their dry weight.

6. The feed or food product composition as in claim 1, which has a bound water content of less than 5 wt%.

7. The feed or food product composition as in claim 1, which has a free water content of not more than 10 times its dry weight.

8. The feed or food product composition as in claim 7, wherein the pH of said free water is between 5 and 7.

9. The feed or food product composition as in claim 1, which further comprises additional pre-separated colostrum fat.

10. The feed or food product composition as in claim 1, which further comprises vegetable fat.

11. The feed or food product composition as in claim 10, wherein said vegetable fat is rice, soy, rape, palm, or maize fat, or a mixture thereof.

12. The feed or food product composition as in claim 1, which has a fat content between 5 and 20 wt% on dry weight basis.

13. The feed or food product composition as in claim 1, wherein said bioactive bovine colostrum components are peptides, growth factors, immunoglobulins, lactoferrin, or lactoperoxidase.

14. The feed or food product composition as in claim 13, wherein said bioactive bovine colostrum components comprise 10-40 wt% bovine colostrum powder of 20-25 wt% IgG₁ on dry weight basis.

15. The feed or food product composition as in claim 1, which further comprises nutritionally effective amounts of non-colostrum proteins, carbohydrates, vitamins and minerals.

16. The feed or food product composition as in claim 15, wherein said non-colostrum proteins are whey proteins from bovine milk.

17. The feed or food product composition as in claim 15, wherein said non-colostrum carbohydrates are plant polysaccharides and low molecular weight carbohydrates.

## Patentansprüche

1. Futter- oder Nahrungsmittelzusammensetzung zur Abgabe an den Verdauungstrakt eines Säugers, die eine Mischung aus bovinem Kolostrum enthält, mit bioaktiven Komponenten, und organische Feinpartikel mit einer Größe zwischen 0,3 und 7 mm Durchmesser.

2. Futter- oder Nahrungsmittelzusammensetzung nach Anspruch 1, wobei der Gehalt der organischen Feinpartikel zwischen 40 und 80 Gewichts% auf Basis des Trockengewichts liegt.

3. Futter- oder Nahrungsmittelzusammensetzung nach Anspruch 1, wobei die organischen Feinpartikel aus feinen unlöslichen diätetischen Fasern besteht.

4. Futter- oder Nahrungsmittelzusammensetzung nach Anspruch 3, wobei es sich bei den feinen unlöslichen diätetischen Fasern um Feinpartikel von Zellulose, Reis, Mais oder Soja, oder einer Mischung daraus handelt.

5. Futter- oder Nahrungsmittelzusammensetzung nach Anspruch 3, wobei die feinen unlöslichen diätetischen Fasern ein Wasserspeichervermögen des bis zu 10-fachen ihres Trockengewichts aufweisen.

6. Futter- oder Nahrungsmittelzusammensetzung nach Anspruch 1, deren Gehalt an gebundenem Wasser weniger als 5 Gewichts% beträgt.

7. Futter- oder Nahrungsmittelzusammensetzung nach Anspruch 1, deren Gehalt an nichtgebundenem Wasser nicht mehr als das 10-fache ihres Trockengewichts ausmacht.

8. Futter- oder Nahrungsmittelzusammensetzung nach Anspruch 7, wobei der pH-Wert des nichtgebundenen Wassers zwischen 5 und 7 liegt.

9. Futter- oder Nahrungsmittelzusammensetzung nach Anspruch 1, die außerdem zusätzliches vorsepariertes Kolostrumfett enthält.

10. Futter- oder Nahrungsmittelzusammensetzung nach Anspruch 1, die außerdem Pflanzenfett enthält.

11. Futter- oder Nahrungsmittelzusammensetzung nach Anspruch 10, wobei es sich bei dem Pflanzenfett um Reis-, Soja-, Raps-, Palmen- oder Maisfett oder einer Mischung daraus handelt.

12. Futter- oder Nahrungsmittelzusammensetzung nach Anspruch 1, die einen Fettgehalt zwischen 5 und 20 Gewichts% auf Basis des Trockengewichts aufweist.

13. Futter- oder Nahrungsmittelzusammensetzung nach Anspruch 1, wobei es sich bei den bioaktiven Komponenten des bovinen Kolostrums um Peptide, Wachstumsförderer, Immunoglobuline, Lactoferrin oder Lactoperoxidase handelt.

14. Futter- oder Nahrungsmittelzusammensetzung nach Anspruch 13, wobei die bioaktiven Komponenten des bovinen Kolostrums 10-40 Gewichts% bovines Kolostrumpulver aus 20-25 Gewichts% IgG₁ auf Basis des Trockengewichts enthalten.

15. Futter- oder Nahrungsmittelzusammensetzung nach Anspruch 1, die außerdem nutritionell wirksame Mengen von nicht-Kolostrum Proteinen, Kohlenhydraten, Vitaminen und Mineralien enthält.

16. Futter- oder Nahrungsmittelzusammensetzung nach Anspruch 15, wobei es sich bei den nicht-Kolostrum Proteinen um Molkeproteine aus Kuhmilch handelt.

17. Futter- oder Nahrungsmittelzusammensetzung nach Anspruch 15, wobei es sich bei den nicht-Kolostrum Kohlenhydraten um pflanzliche Polysaccharide und Kohlenhydrate mit niedrigem Molekulargewicht handelt.

## Revendications

1. Composition d'aliment ou de produit alimentaire destinée à être délivrée au tractus digestif d'un mammifère, qui comprend un mélange de colostrum bovin, comprenant des composants bioactifs, et une matière particulaire organique ayant une taille comprise entre 0,3 et 7 mm de diamètre.

2. Composition d'aliment pour animaux ou de produit alimentaire selon la revendication 1, dans laquelle la teneur en ladite matière particulaire organique est comprise entre 40 et 80 % en poids sur la base du poids sec.

3. Composition d'aliment pour animaux ou de produit alimentaire selon la revendication 1, dans laquelle ladite matière particulaire organique est des fibres alimentaires, particulaires et insolubles.

4. Composition d'aliment pour animaux ou de produit alimentaire selon la revendication 3, dans laquelle lesdites fibres alimentaires, particulaires et insolubles sont des fibres particulaires de cellulose, de riz, de maïs, ou de soja, ou un mélange de celles-ci.

5. Composition d'aliment pour animaux ou de produit alimentaire selon la revendication 3, dans laquelle lesdites fibres alimentaires, particulaires et insolubles ont une capacité de rétention d'eau d'au plus 10 fois leur poids sec.

6. Composition d'aliment pour animaux ou de produit alimentaire selon la revendication 1, qui a une teneur en eau liée inférieure à 5 % en poids.

7. Composition d'aliment pour animaux ou de produit alimentaire selon la revendication 1, qui a une teneur en eau libre n'excédant pas 10 fois son poids sec.

8. Composition d'aliment pour animaux ou de produit alimentaire selon la revendication 7, dans laquelle le pH de ladite eau libre est compris entre 5 et 7.

9. Composition d'aliment pour animaux ou de produit alimentaire selon la revendication 1, qui comprend en outre de la matière grasse du colostrum pré-séparée supplémentaire.

10. Composition d'aliment pour animaux ou de produit alimentaire selon la revendication 1, qui comprend en outre de la matière grasse végétale.

11. Composition d'aliment pour animaux ou de produit alimentaire selon la revendication 10, dans laquelle ladite matière grasse végétale est de la matière grasse de riz, de soja, de colza, de palme, ou de maïs, ou un mélange de celles-ci.

12. Composition d'aliment pour animaux ou de produit alimentaire selon la revendication 1, qui a une teneur en matière grasse comprise entre 5 et 20 % en poids sur la base du poids sec.

13. Composition d'aliment pour animaux ou de produit alimentaire selon la revendication 1, dans laquelle lesdits composants bioactifs de colostrum bovin sont des peptides, des facteurs de croissance, des immunoglobulines, la lactoferrine, ou la lactoperoxydase.

14. Composition d'aliment pour animaux ou de produit alimentaire selon la revendication 13, dans laquelle lesdits composants bioactifs de colostrum bovin comprennent de 10 à 40 % en poids de poudre de colostrum bovin ayant de 20 à 25 % en poids d'IgG₁ sur la base du poids sec.

15. Composition d'aliment pour animaux ou de produit alimentaire selon la revendication 1, comprenant en outre des quantités efficaces sur le plan nutritionnel de protéines, d'hydrates de carbone, de vitamines et de minéraux ne provenant pas du colostrum.

16. Composition d'aliment pour animaux ou de produit alimentaire selon la revendication 15, dans laquelle lesdites protéines ne provenant pas du colostrum sont des protéines du lactosérum de lait bovin.

17. Composition d'aliment pour animaux ou de produit alimentaire selon la revendication 15, dans laquelle lesdits hydrates de carbone ne provenant pas du colostrum sont des polysaccharides végétaux et des hydrates de carbone de faible masse moléculaire.
